# EUROPEAN PATENT APPLICATION

(11) **EP 4 471 007 A1**
(43) Date of publication of application: **04.12.2024**
(21) Application number: 23747031.5
(22) Date of filing: 26.01.2023
(51) Int. Cl.: C07C 273/04, C07C 275/00

(54) **UREA SYNTHESIS METHOD**

(30) Priority: 26.01.2022 JP 2022010023
(71) Applicant: Toyo Engineering Corporation, Tokyo 105-0003 (JP)
(72) Inventor: YANAGAWA, Takahiro, Narashino-shi, Chiba 275-0024 (JP); KOJIMA, Yasuhiko, Narashino-shi, Chiba 275-0024 (JP); SANO, Keiji, Narashino-shi, Chiba 275-0024 (JP)
(74) Representative: Gille Hrabal Partnerschaftsgesellschaft mbB Patentanwälte
(86) International application number: PCT/JP2023/002435
(87) International publication number: WO 2023/145821

(57) **Abstract**

Disclosed is a urea synthesis method including, bringing a urea synthesis liquid produced in a urea synthesis tower A into contact with at least part of raw material carbon dioxide under heating in a stripper C, introducing a separated mixed gas into a condenser B to condense it, and circulating the condensate to the urea synthesis tower A, wherein an oxygen feed concentration with respect to the raw material carbon dioxide is 100 to 2,000 ppm, and in the urea synthesis tower A, a urea synthesis pressure is 125 to 145 bar, a urea synthesis temperature is 175°C to 190°C, a N/C is 3.5 to 4.0, and a H/C is 0.70 or less.

## Description

### Technical Field

The present invention relates to a method for synthesizing urea from ammonia and carbon dioxide while forming a passivation film with oxygen on a metal surface inside an apparatus, the urea synthesis method being capable of reducing energy or running costs required for urea synthesis.

### Background Art

Urea is an important raw material for nitrogen-based fertilizers, one of the three major elements essential for the growth of plants, and it has supported food production in the world from old times. Even now, there is a demand for increased fertilizer production corresponding to increased food production associated with population growth in emerging countries of the world, and construction of a urea synthesis apparatus based on a urea synthesis method excellent in reliability and productivity is an important issue.

Here, the reliability mainly refers to the property of protecting a metal surface inside this apparatus from highly metal-corrosive ammonium carbamate produced as a reaction intermediate when urea is synthesized from ammonia and carbon dioxide. On the other hand, the productivity refers to not only increasing a yield of the reaction of ammonia and carbon dioxide, but also a reduction in total urea production costs including a reduction in apparatus construction costs and a reduction in running costs under improved operating conditions.

Technical developments for increasing such reliability and productivity have been approached mainly from three viewpoints. The first is a chemical viewpoint that a passivation film is formed on a metal surface of an apparatus to increase corrosion resistance. The second is a material viewpoint that resistance to corrosion by ammonium carbamate is increased by improvements in a material of an apparatus itself. The third is a process viewpoint that improvements in corrosion resistance and a reaction yield are attempted by improvements in a production process or an apparatus. However, these are in a conflicting relationship with one another, and a balanced urea synthesis method is required even now.

In other words, in one method, oxygen is mixed into carbon dioxide or ammonia, a starting raw material, such that about 1 to 3 nm of a passivation film made of a hydroxide of Fe or Cr or an oxide of Cr is formed at a high temperature and a high pressure.

However, feeding an excess of oxygen as an oxidant increases inert gases, as well as increasing a H/C (molar ratio of water to carbon dioxide) described later, resulting in a reduced reaction yield. On the other hand, a scrubber for finally separating and removing oxygen from unconverted raw materials or other volatile components, or a hydrogen combustion removal device for preventing the risk of a trace amount of hydrogen contained in a urea product being mixed with oxygen is also required. Thus, while oxygen increases corrosion resistance as its feed amount increases, its adverse effect on running costs is a problem.

Therefore, developments have been advanced toward increased corrosion resistance of stainless steel itself. For example, as disclosed in Patent Literature 1, an austenitic-ferritic duplex stainless steel improved by paying attention to effects of Cr, Ni, Mo and N and having a Cr content of 26 mass% or more and less than 28 mass%, a Ni content of 6 to 10 mass%, a Mo content of 0.2 to 1.7 mass%, and a N content of more than 0.3 mass% and 0.4 mass% or less has been proposed.

However, there is a problem that a high material price of such a highly corrosion-resistant austenitic-ferritic duplex stainless steel with a high Cr content leads to increased construction costs of a urea plant, and it is thus difficult to construct a urea synthesis apparatus attaining both reliability and productivity.

Therefore, as disclosed in Patent Literature 2, improvements in a production process or an apparatus have also been performed. Particularly, introducing a stripper to effectively recycle unreacted products or providing a condenser with a urea synthesis function in the same manner as in a reactor can increase a reaction yield or downsize production facilities, and may contribute to increased productivity.

However, there is a room for improvement in these techniques in terms of a urea synthesis method that satisfies reliability and productivity with a good balance of a concentration of oxygen required for corrosion resistance, a stainless steel material, and an efficient production process.

Therefore, as disclosed in Patent Literature 3, a urea synthesis method in which oxygen fed as an oxidant (hereinafter described as corrosion prevention oxygen) is the required minimum and the anticorrosiveness is excellent though inexpensive general-purpose stainless steel is selected, and furthermore, urea can be produced at high reaction yield, low construction cost, and low cost has been proposed.

However, further improvements are required of the conventional urea synthesis methods in terms of energy or running costs required for urea synthesis.

### Prior Art Documents

### Patent Literatures

Patent Literature 1: JP-A 2003-301241
Patent Literature 2: JP-A H10-182587
Patent Literature 3: WO-A 2014/192823

### Summary of the Invention

### Problem to be Solved by the Invention

The present invention has been made to solve the problems of the conventional techniques explained above. In other words, an object of the present invention is to provide a method for synthesizing urea from ammonia and carbon dioxide while forming a passivation film with oxygen on a metal surface inside an apparatus, the urea synthesis method being capable of reducing energy or running costs required for urea synthesis.

### Means to Solve the Problem

As a result of conducting intensive studies to solve the above problem, the present inventors found that reducing a urea synthesis pressure in a urea synthesis tower is very effective in reducing energy or running costs required for urea synthesis, and led to the completion of the present invention.

The present invention is a urea synthesis method including, reacting ammonia with carbon dioxide at a urea synthesis temperature and a urea synthesis pressure in a urea synthesis tower, bringing a urea synthesis liquid containing at least produced urea, unreacted ammonia, unreacted carbon dioxide and water into contact with at least part of the raw material carbon dioxide under heating at a pressure substantially equal to the urea synthesis pressure in a stripper to separate the unreacted ammonia and the unreacted carbon dioxide as a mixed gas of ammonia, carbon dioxide and water, further treating the urea synthesis liquid containing unseparated unreacted ammonia and unreacted carbon dioxide to obtain urea, while introducing the mixed gas separated in the stripper into a bottom of a condenser to bring the mixed gas into contact with an absorbing medium under cooling to thereby condense the mixed gas, and circulating the thus obtained condensate to the urea synthesis tower, characterized in that
an oxygen feed concentration with respect to the raw material carbon dioxide is 100 to 2,000 ppm,
the urea synthesis pressure in the urea synthesis tower is 125 to 145 bar,
the urea synthesis temperature in the urea synthesis tower is 175°C to 190°C,
a N/C (molar ratio of ammonia to carbon dioxide) in the urea synthesis tower is 3.5 to 4.0, and
a H/C (molar ratio of water to carbon dioxide) in the urea synthesis tower is 0.70 or less.

Further, the present invention is a method for improving an existing urea synthesis apparatus to reduce a urea synthesis pressure in a urea synthesis tower, wherein
the existing urea synthesis apparatus is a urea synthesis apparatus for reacting ammonia with carbon dioxide at a urea synthesis temperature and a urea synthesis pressure in a urea synthesis tower, bringing a urea synthesis liquid containing at least produced urea, unreacted ammonia, unreacted carbon dioxide and water into contact with at least part of the raw material carbon dioxide under heating at a pressure substantially equal to the urea synthesis pressure in a stripper to separate the unreacted ammonia and the unreacted carbon dioxide as a mixed gas of ammonia, carbon dioxide and water, further treating the urea synthesis liquid containing unseparated unreacted ammonia and unreacted carbon dioxide to obtain urea, while introducing the mixed gas separated in the stripper into a bottom of a condenser to bring the mixed gas into contact with an absorbing medium under cooling to thereby condense the mixed gas, and circulating the thus obtained condensate to the urea synthesis tower, and
a material of piping connecting the stripper and the condenser in the existing urea synthesis apparatus is neither of the materials (a) and (b) below,
the method including, replacing the piping connecting the stripper and the condenser in the existing urea synthesis apparatus with piping including the material (a) or (b) below to enable reduction of the urea synthesis pressure in the urea synthesis tower,
   (a) a general-purpose austenitic-ferritic duplex stainless steel with a Cr content of 21 to 26 mass%, a Ni content of 4.5 to 7.5 mass%, a Mo content of 2.5 to 3.5 mass%, a N content of 0.08 to 0.30 mass%, a C content of 0.03 mass% or less, a Si content of 1.0 mass% or less, a Mn content of 2.0 mass% or less, a P content of 0.04 mass% or less, and a S content of 0.03 mass% or less, and
   (b) an austenitic-ferritic duplex stainless steel with a Cr content of 26 to 35 mass%, a Ni content of 3 to 10 mass%, a Mo content of 1.0 to 4.0 mass%, and a N content of 0.2 to 0.6 mass%.

Further, the present invention is a method for improving an existing urea synthesis apparatus to reduce a urea synthesis pressure in a urea synthesis zone, wherein
the existing urea synthesis apparatus is a urea synthesis apparatus for reacting ammonia with carbon dioxide at a urea synthesis temperature and a urea synthesis pressure in a urea synthesis zone, bringing a urea synthesis liquid containing at least produced urea, unreacted ammonia, unreacted carbon dioxide and water into contact with at least part of the raw material carbon dioxide under heating at a pressure substantially equal to the urea synthesis pressure in a stripper to separate the unreacted ammonia and the unreacted carbon dioxide as a mixed gas of ammonia, carbon dioxide and water, further treating the urea synthesis liquid containing unseparated unreacted ammonia and unreacted carbon dioxide to obtain urea, while introducing the mixed gas separated in the stripper into a bottom of a condensation zone to bring the mixed gas into contact with an absorbing medium under cooling to thereby condense the mixed gas, and circulating the thus obtained condensate to the urea synthesis zone,
the urea synthesis zone and the condensation zone are present in the same container or in separate containers,
the condensation zone is present in a horizontal container, and
a material of piping connecting the stripper and the condensation zone in the existing urea synthesis apparatus is neither of the materials (a) and (b) below,
the method including, replacing the piping connecting the stripper and the condensation zone in the existing urea synthesis apparatus with piping including the material (a) or (b) below to enable reduction of the urea synthesis pressure in the urea synthesis zone,
   (a) a general-purpose austenitic-ferritic duplex stainless steel with a Cr content of 21 to 26 mass%, a Ni content of 4.5 to 7.5 mass%, a Mo content of 2.5 to 3.5 mass%, a N content of 0.08 to 0.30 mass%, a C content of 0.03 mass% or less, a Si content of 1.0 mass% or less, a Mn content of 2.0 mass% or less, a P content of 0.04 mass% or less, and a S content of 0.03 mass% or less, and
   (b) an austenitic-ferritic duplex stainless steel with a Cr content of 26 to 35 mass%, a Ni content of 3 to 10 mass%, a Mo content of 1.0 to 4.0 mass%, and a N content of 0.2 to 0.6 mass%.

### Effects of the Invention

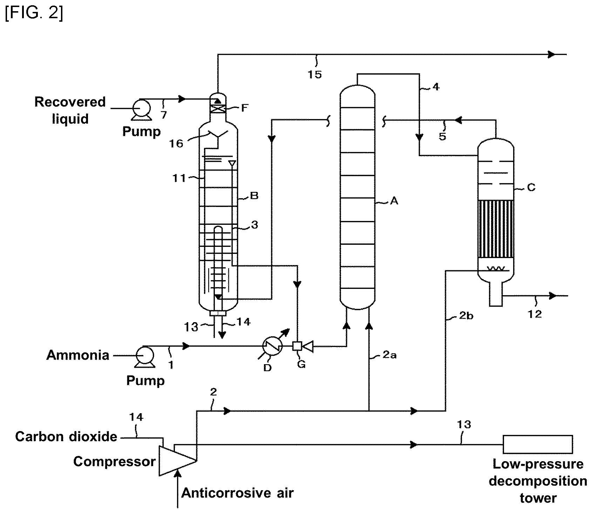

The present invention enables reduction of a urea synthesis pressure in a urea synthesis tower particularly by reducing an oxygen feed concentration with respect to raw material carbon dioxide, and as a result, enables reduction of energy required for urea synthesis, as well as enabling reduction of running costs. Note that, in the present invention, an "oxygen feed concentration with respect to raw material carbon dioxide" means a concentration of oxygen (which is oxygen in air if air is fed) fed to a urea synthesis apparatus as an oxidant with respect to carbon dioxide fed to the urea synthesis apparatus as a raw material, and for example, in the case of FIG. 2, it means a concentration of oxygen with respect to carbon dioxide in a line 2.

Specifically, reducing an oxygen feed concentration with respect to raw material carbon dioxide means reducing a feed amount of oxygen (which is air if air containing oxygen is fed), and this increases a proportion of a raw material gas (raw material carbon dioxide) in a mixed gas, and decreases a partial pressure of gases other than the raw material (inert gases) in a urea synthesis step, thus enabling reduction of a urea synthesis pressure. Note that the idea of reducing a urea synthesis pressure by such a technique is an idea that had never existed in the past. This is because it had been considered that optimum urea synthesis pressures vary depending on conditions such as, for example, a N/C and others, and it had not been known that a urea synthesis pressure can be reduced by simply reducing an oxygen feed concentration, and urea synthesis can be performed satisfactorily even at such a low pressure.

### Brief Description of the Drawings

[FIG. 1] A process flow of a urea synthesis apparatus using a urea synthesis method in carbon dioxide stripping mode of the present invention.
[FIG. 2] An embodiment of a urea synthesis apparatus using the urea synthesis method in carbon dioxide stripping mode of the present invention, and a process flow of a urea synthesis method used in example 1 and reference example 1.

### Embodiments of the Invention

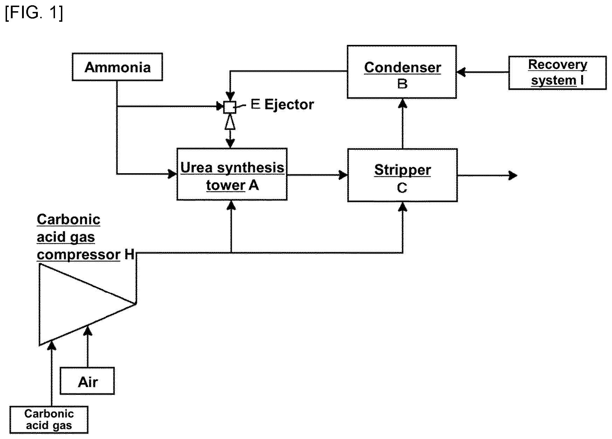

One embodiment of the present invention is suitably a urea synthesis method in carbon dioxide stripping mode shown in FIG. 1, in other words, a method in which raw material liquid ammonia is fed to a urea synthesis tower A, and corrosion prevention oxygen is fed as air together with raw material gaseous carbon dioxide (carbonic acid gas in FIG. 1) to the urea synthesis tower A and a stripper C. Particularly, embodiments suitable as the urea synthesis method are various synthesis processes disclosed in JP-A H10-182587, and one embodiment thereof is shown in FIG. 2, but the urea synthesis method is not limited thereto.

For example, a form in which raw material liquid ammonia is fed to the urea synthesis tower A and a condenser B, and raw material gaseous carbon dioxide containing anticorrosive air fed from a carbonic acid gas compressor H is not fed to the urea synthesis tower A, but fed to the stripper C alone may be possible. Further, for example, a form in which raw material liquid ammonia is fed to the urea synthesis tower A alone, and raw material gaseous carbon dioxide containing corrosion prevention oxygen fed from the carbonic acid gas compressor H is not fed to the stripper C, but fed to the urea synthesis tower A alone (ammonia stripping mode) may be possible. However, it should be noted that, as shown in the conventional art, pressure and temperature conditions and compositions of retained and passing fluids in regions such as the urea synthesis tower A, the stripper C, the condenser B and others vary with different urea synthesis methods, and thus, amounts of corrosion prevention oxygen required by stainless steels applied to the modes are different.

Further, in the process flow of FIG. 1, an ejector G is used for the purpose of pressurizing a liquid from the condenser B to circulate a synthesis liquid from the viewpoints of a simple structure and excellent durability and maintainability, but another pressurizing unit such as a pump or the like can also be used for circulation. However, this may be omitted if circulation of the urea synthesis liquid can be achieved satisfactorily by an arrangement of devices. For example, the urea synthesis liquid can be circulated only by gravity if the devices are arranged ingeniously. However, providing a pressurizing unit such as the ejector G or the like enables installation of the urea synthesis tower A, the condenser B and the stripper C at low positions, and thus is preferable in terms of installation work or maintenance. Further, the above devices may be installed separately from one another, or the condenser B may be combined with a scrubber or the like.

On the other hand, an orientation of installation of the condenser may be horizontal or vertical. For example, installing a horizontal condenser on the ground or at a position close to the ground facilitates installation work or maintenance of the urea synthesis tower in combination with its low overall height. Further, using a vertical condenser not only enables savings in installation area, but also enables gas-liquid countercurrent contact and a sufficiently long residence time in the condensation portion.

Further, a urea production apparatus used in the urea synthesis method of the present invention may be a new apparatus newly constructed in its entirety or a modified apparatus (so-called revamping) formed by adding a portion to existing facilities or replacing a portion thereof.

In such a urea synthesis apparatus, portions of the urea synthesis tower A, the stripper C and the condenser B and piping connecting them coming in contact with corrosive fluids can be at least partially made of an austenitic-ferritic duplex stainless steel of the composition (a) below.

### [Composition (a) of austenitic-ferritic duplex stainless steel applied to the present invention]

Cr: 21 to 26 mass%
Ni: 4.5 to 7.5 mass%
Mo: 2.5 to 3.5 mass%
N: 0.08 to 0.30 mass%
C: 0.03 mass% or less
Si: 1.0 mass% or less
Mn: 2.0 mass% or less
P: 0.04 mass% or less
S: 0.03 mass% or less

The remainder are Fe, and for example, impurities, additives or the like.

Particularly, it is preferable to use S31803 or S31260 available at low prices as a general-purpose austenitic-ferritic duplex stainless steel.

Further, it is more preferable to use an austenitic-ferritic duplex stainless steel of the composition (a1) below as such an austenitic-ferritic duplex stainless steel.

### [Composition (a1) of austenitic-ferritic duplex stainless steel applied to the present invention]

Cr: 24 to 26 mass%
Ni: 5.5 to 7.5 mass%
Mo: 2.5 to 3.5 mass%
N: 0.08 to 0.30 mass%
C: 0.03 mass% or less
Si: 1.0 mass% or less
Mn: 1.5 mass% or less
P: 0.04 mass% or less
S: 0.03 mass% or less

The remainder are Fe, and for example, impurities, additives or the like.

Particularly, it is preferable to use S31260 available at low prices as a general-purpose austenitic-ferritic duplex stainless steel.

Further, it is preferable to use an austenitic-ferritic duplex stainless steel of the composition (b) below in the stripper where corrosion is more likely to proceed.

### [Composition (b) of austenitic-ferritic duplex stainless steel applied to the present invention]

Cr: 26 to 35 mass%
Ni: 3 to 10 mass%
Mo: 1.0 to 4.0 mass%
N: 0.2 to 0.6 mass%

The remainder are Fe, and for example, impurities, additives or the like.

### (Note that the Cr content may be more than 26 mass% and 35 mass% or less.)

Particularly, it is preferable to use, for example, S32707 or S32808 as such an austenitic-ferritic duplex stainless steel.

Further, S31603 general-purpose stainless steel can also be used according to corrosion environments such as piping, valves or the like, and an attempt to further reduce facility costs without increasing an amount of corrosion prevention oxygen can thus be also attained.

Examples of a specific preferable aspect using each of the above stainless steels include, for example,
an aspect in which a material of at least one device selected from the group consisting of the urea synthesis tower, the stripper, the condenser and piping connecting at least two of the urea synthesis tower, the stripper and the condenser is a general-purpose austenitic-ferritic duplex stainless steel of the above composition (a) or an austenitic-ferritic duplex stainless steel of the above composition (b),
an aspect in which a material of the stripper is a general-purpose austenitic-ferritic duplex stainless steel of the above composition (a) or an austenitic-ferritic duplex stainless steel of the above composition (b),
an aspect in which a material of piping connecting the stripper and the condenser is a general-purpose austenitic-ferritic duplex stainless steel of the above composition (a) or an austenitic-ferritic duplex stainless steel of the above composition (b), and others.

The urea synthesis apparatus made of such materials as above is preferably operated under the following operating conditions of the urea synthesis tower A: a pressure of 125 to 145 bar, a N/C of 3.5 to 4.0, a H/C of 1.0 or less, a residence time of 10 to 40 minutes, and a temperature of 175°C to 190°C, the following operating conditions of the stripper C: a pressure of 125 to 145 bar and particularly preferably 130 to 140 bar, and a temperature of 170 to 190°C, and the following operating conditions of the condenser B: a pressure of 125 to 145 bar, a temperature of 130 to 250°C and particularly preferably 170 to 190°C, a N/C of 2.5 to 3.5 and particularly preferably 2.7 to 3.3, a H/C of 1.0 or less, and a residence time of 10 to 30 minutes, mainly from the viewpoint of a reaction yield. Here, a N/C is a molar ratio of ammonia (including ammonia converted to ammonium carbamate and urea) to carbon dioxide (including carbon dioxide converted to ammonium carbamate and urea) (hereinafter referred to as "N/C"), and a H/C is a molar ratio of water (excluding water produced in the urea synthesis reaction) to carbon dioxide (including carbon dioxide converted to ammonium carbamate and urea) (hereinafter referred to as "H/C").

Such operating conditions are preferable on the basis of the fact that the reactions of the formulas 1 and 2 below not only take place in the urea synthesis tower A, but also proceed in the condenser B while condensing ammonia gas and/or carbon dioxide gas, as shown in FIG. 1. Note that the formula 1 represents a reaction in which ammonium carbamate (NH₂CO₂NH₄) is produced by a reaction of ammonia (NH₃) and carbon dioxide (CO₂). The formula 2 represents an equilibrium reaction in which urea (CO(NH₂)₂) is produced by a dehydration reaction of ammonium carbamate (NH₂CO₂NH₄).

2NH₃ + CO₂ → NH₂CO₂NH₄ (exothermic reaction) formula 1

NH₂CO₂NH₄ ↔ CO(NH₂)₂ + H₂O (endothermic reaction) formula 2

In other words, in FIG. 1, while raw material liquid ammonia is pressurized to a desired pressure by an ammonia pump (not shown) and fed to the urea synthesis tower A, and part thereof is heated by a heat exchanger and also fed to the ejector G, a urea synthesis liquid is also fed to the ejector G from the condenser B, and the urea synthesis liquid containing ammonia from this ejector G is pressurized and fed to the urea synthesis tower A. Raw material gaseous carbon dioxide is pressurized to a desired pressure by the carbonic acid gas compressor H, and most thereof is fed to the stripper C. The remainder of the carbon dioxide is partially fed to the urea synthesis tower A for the purpose of controlling a temperature of the urea synthesis tower A and feeding corrosion prevention oxygen. Here, feeding "most" of the gaseous carbon dioxide to the stripper C specifically means that a ratio [X_{C}/X_{A}] of an amount of the raw material carbon dioxide fed to the stripper C [Xc (t/d)] (e.g., an amount of raw material carbon dioxide fed through a line 2b in FIG. 2) to an amount of the raw material carbon dioxide fed to the urea synthesis tower A [X_{A} (t/d)] (e.g., an amount of raw material carbon dioxide fed through a line 2a in FIG. 2) is about 90/10 to 70/30. Note that, for example, as shown in FIGS. 1 and 2, if most of the raw material carbon dioxide mixed in advance with the anticorrosive air (which may be a gas other than air containing corrosion prevention oxygen) is fed to the stripper C, most of the corrosion prevention oxygen is also fed to the stripper C, and thus, corrosion of the stripper C relatively susceptible to corrosion can be prevented effectively. Air is usually fed as the corrosion prevention oxygen to a suction side at a first stage or an intermediate stage of the carbonic acid gas compressor H. Urea is synthesized in the urea synthesis tower A and the condenser B, and an effluent containing urea out of the urea synthesis tower A is fed to the stripper C. In this effluent, synthesized urea, water, ammonium carbamate and unreacted ammonia are present as a liquid phase, and part of unreacted ammonia and carbon dioxide is present as a gas phase together with inert gases. Here, the inert gases collectively refer to impurities, such as hydrogen, nitrogen, and others, contained in the anticorrosive air introduced for preventing corrosion of the urea synthesis apparatus configured of, for example, the urea synthesis tower A, the stripper C, the condenser B, a scrubber and piping connecting them or the like, and in the raw material carbon dioxide. The synthesis liquid from the urea synthesis tower A is fed to the stripper C, and the unreacted ammonia and unreacted carbon dioxide are treated therein. The raw material carbon dioxide is used as a stripping agent.

Accordingly, the operating conditions of the urea synthesis tower A, the stripper C and the condenser B in such a production apparatus as shown in FIG. 1 are each determined by the reasons described below including Le Chatelier's principle.

A pressure inside the urea synthesis tower A (urea synthesis pressure) is 125 to 145 bar and preferably 130 to 140 bar. If this synthesis pressure is 125 bar or more, an operating pressure providing a margin against a synthesis equilibrium pressure at a temperature preferable for urea synthesis (e.g., 180°C or more) can be adopted and a reduction in a reaction yield due to gasification can also be prevented due to a reduced partial pressure of inert gases. Further, if the synthesis pressure is 145 bar or less, energy for pressurizing the raw material ammonia, the raw material carbonic acid gas and an unreacted ammonium carbamate liquid can be significantly suppressed, and running costs can be reduced. Here, the ammonium carbamate liquid is a liquid obtained by recovering the unreacted ammonia and carbon dioxide as an aqueous ammonium carbamate solution in a recovery step downstream from the synthesis step.

Next, a synthesis temperature inside the urea synthesis tower A is 175°C to 190°C as the higher it is, the more the reaction of the formula 1 proceeds to the left side and the reaction of the formula 2 proceeds to the right side, and thus, there is a moderate region in terms of a reaction yield. If the synthesis temperature is 175°C or more, a decrease in a reaction rate of urea production can be prevented. Further, if the synthesis temperature is 190°C or less, an increase in the risk of the so-called active corrosion, in addition to an increase in a corrosion rate, can be prevented. This synthesis temperature can be controlled by, for example, a preheating temperature of ammonia driving the ejector G and/or the amount of carbon dioxide fed to the urea synthesis tower A.

A N/C inside the urea synthesis tower A is stoichiometrically 2 according to the formula 1, but is 3.5 to 4.0 in the present invention as a state where an excess of unreacted ammonia is present is preferable. It is more preferably 3.6 to 3.8.

A H/C inside the urea synthesis tower A is 0.70 or less in the present invention as a lower H/C is better from the viewpoint of a reaction yield according to the formulas 1 and 2, and thus, an amount of water fed to a recovery apparatus is preferably the bare minimum. It is more preferably 0.6 or less. This H/C is more preferably the lowest possible one, but the H/C is often determined on the basis of an amount of water required for the absorption of unreacted substances (ammonia and carbon dioxide) out of the urea synthesis apparatus in a recovery apparatus for recovering them (not shown), and actually, some water is present. It is unnecessary to set a lower limit of the H/C.

A residence time of the urea synthesis liquid inside the urea synthesis tower A is preferably 10 to 40 minutes. If this residence time is 10 minutes or more, the progress of the urea synthesis reaction is accelerated. On the other hand, if the residence time is more than 40 minutes, a reaction yield close to an equilibrium reaction yield is already reached, and thus, a further increase thereof can be hardly expected.

Under the above operating conditions, a reaction yield of about 60% or more and 75% or less is achieved inside the urea synthesis tower A. Here, a reaction yield based on carbon dioxide is a ratio of a molar number of carbon dioxide converted to urea, of carbon dioxide fed to a device or a zone of interest, to a molar number of the fed carbon dioxide, and is usually expressed in %.

In the operating conditions of the stripper C required to accelerate the reversible reaction of the formula 1, basically, a higher temperature and a lower pressure are preferable, but in view of a balance in the entire urea synthesis apparatus, a pressure is 125 to 145 bar and particularly preferably 130 to 140 bar, and a temperature is 170 to 190°C. Note that stripping is performed at a pressure substantially equal to the urea synthesis pressure in the stripper C. Here, "pressure substantially equal" means that a difference between the pressure and the urea synthesis pressure is within ±5 bar.

The operating conditions of the condenser B can be considered in the same manner as in the urea synthesis tower A as synthesizing capability is imparted thereto, and a reaction yield of 20 to 60% can be achieved under substantially the same conditions as those of the urea synthesis tower A, i.e., a pressure of 125 to 145 bar, a temperature of 130 to 250°C and particularly preferably 170 to 190°C, a N/C of 2.5 to 3.5 and particularly preferably 2.7 to 3.3, a H/C of 0.7 or less, and a residence time of 10 to 30 minutes.

In such a synthesis apparatus and under such operating conditions, corrosion prevention oxygen for devices is fed as air concurrently with carbon dioxide, as shown in FIG. 1. Amounts of corrosion prevention oxygen required for the urea synthesis tower A, the stripper C and the condenser B vary from device to device, and the stripper C is in the most corrosive environment, followed by the condenser B and the urea synthesis tower A in this order. However, in the synthesis apparatus shown in FIG.1, an oxygen feed amount is determined on the basis of the bare minimum for the stripper C as the corrosion prevention oxygen is distributed throughout the synthesis apparatus, and preferably 100 to 2,000 ppm and further preferably 100 to 1,000 ppm of corrosion prevention oxygen is required. Further, if a stainless steel material used for each portion of the synthesis apparatus is selected, operation can be performed in a further preferable amount of 100 to 500 ppm of corrosion prevention oxygen. However, if only general-purpose austenitic-ferritic duplex stainless steels are used, each lower limit above is preferably 150 ppm or more.

The oxygen feed concentration with respect to the carbon dioxide can be reduced to 100 to 500 ppm if such an austenitic-ferritic duplex stainless steel as have a Cr content of 26 to 35 mass%, a Ni content of 3 to 10 mass%, a Mo content of 1.0 to 4.0 mass% and a N content of 0.2 to 0.6 mass%, for example, S32707 or S32808, is used particularly for the stripper where corrosion is more likely to proceed and the piping connecting the stripper and the condenser.

A method for feeding this corrosion prevention oxygen is preferably feeding by mixing anticorrosive air into raw material carbon dioxide, as shown in the process flow in FIG. 1, from the viewpoint of being unnecessary to newly dispose a pressurizing device. However, the present invention is not limited to this feeding method. For example, an introduction port for anticorrosive air and a device for performing pressurization to a predetermined pressure may be disposed at each device individually to feed anticorrosive air to each device. Also in this case, the corrosion prevention oxygen is distributed throughout the synthesis apparatus, and thus, the amount of the anticorrosive air to be fed, i.e., the corrosion prevention oxygen concentration remains unchanged.

If the corrosion prevention oxygen is thus reduced, gas composition is outside an explosion range, and a hydrogen combustion removal device can be omitted, but it may be installed for safety. If installed, it is preferably located at a middle stage or an outlet of the carbon dioxide compressor downstream from the anticorrosive air or located upstream from a carbon dioxide introduction stage of the stripper as hydrogen can be removed through catalyst combustion within the hydrogen removal device using part of the introduced corrosion prevention oxygen and it is prior to an explosion range under high temperature conditions as in the stripper.

While the method of the present invention is explained above, the present invention can also be utilized as a method for improving an existing urea synthesis apparatus to reduce a urea synthesis pressure in a urea synthesis tower.

In other words, the present invention is also useful as a method for improving an existing urea synthesis apparatus, wherein
the existing urea synthesis apparatus is a urea synthesis apparatus for reacting ammonia with carbon dioxide at a urea synthesis temperature and a urea synthesis pressure in a urea synthesis tower, bringing a urea synthesis liquid containing at least produced urea, unreacted ammonia, unreacted carbon dioxide and water into contact with at least part of the raw material carbon dioxide under heating at a pressure substantially equal to the urea synthesis pressure in a stripper to separate the unreacted ammonia and the unreacted carbon dioxide as a mixed gas of ammonia, carbon dioxide and water, further treating the urea synthesis liquid containing unseparated unreacted ammonia and unreacted carbon dioxide to obtain urea, while introducing the mixed gas separated in the stripper into a bottom of a condenser to bring the mixed gas into contact with an absorbing medium under cooling to thereby condense the mixed gas, and circulating the thus obtained condensate to the urea synthesis tower, and
a material of piping connecting the stripper and the condenser in the existing urea synthesis apparatus is neither of the materials (a) and (b) below,
the method including, replacing the piping connecting the stripper and the condenser in the existing urea synthesis apparatus with piping including the material (a) or (b) below to enable reduction of the urea synthesis pressure in the urea synthesis tower,
   (a) a general-purpose austenitic-ferritic duplex stainless steel with a Cr content of 21 mass% or more and less than 26 mass%, a Ni content of 4.5 to 7.5 mass%, a Mo content of 2.5 to 3.5 mass%, a N content of 0.08 to 0.30 mass%, a C content of 0.03 mass% or less, a Si content of 1.0 mass% or less, a Mn content of 2.0 mass% or less, a P content of 0.04 mass% or less, and a S content of 0.03 mass% or less, and
   (b) an austenitic-ferritic duplex stainless steel with a Cr content of 26 to 35 mass%, a Ni content of 3 to 10 mass%, a Mo content of 1.0 to 4.0 mass%, and a N content of 0.2 to 0.6 mass%.

Furthermore, the present invention can also be utilized as a method for improving another type of apparatus (an apparatus in which a urea synthesis zone and a condensation zone are present in the same container or in separate containers, and the condensation zone is present in a horizontal container) to reduce a urea synthesis pressure.

In other words, the present invention is also useful as a method for improving an existing urea synthesis apparatus, wherein
the existing urea synthesis apparatus is a urea synthesis apparatus for reacting ammonia with carbon dioxide at a urea synthesis temperature and a urea synthesis pressure in a urea synthesis zone, bringing a urea synthesis liquid containing at least produced urea, unreacted ammonia, unreacted carbon dioxide and water into contact with at least part of the raw material carbon dioxide under heating at a pressure substantially equal to the urea synthesis pressure in a stripper to separate the unreacted ammonia and the unreacted carbon dioxide as a mixed gas of ammonia, carbon dioxide and water, further treating the urea synthesis liquid containing unseparated unreacted ammonia and unreacted carbon dioxide to obtain urea, while introducing the mixed gas separated in the stripper into a bottom of a condensation zone to bring the mixed gas into contact with an absorbing medium under cooling to thereby condense the mixed gas, and circulating the thus obtained condensate to the urea synthesis zone,
the urea synthesis zone and the condensation zone are present in the same container or in separate containers,
the condensation zone is present in a horizontal container, and
a material of piping connecting the stripper and the condensation zone in the existing urea synthesis apparatus is neither of the above materials (a) and (b),
the method including, replacing the piping connecting the stripper and the condensation zone in the existing urea synthesis apparatus with piping including the above material (a) or (b) to enable reduction of the urea synthesis pressure in the urea synthesis zone.

### Examples

Hereinafter, the present invention will be explained more specifically with reference to an example. However, the present invention is not limited by the example.

### <Example 1>

Urea synthesis was performed by a urea synthesis process using a urea synthesis apparatus with a daily production capacity of 2200 tons shown in FIG. 2.

A general-purpose austenitic-ferritic duplex stainless steel was used as a material of piping connecting the stripper C and the condenser B, piping connecting the condenser B and the ejector G, and piping connecting the ejector G and the urea synthesis tower A, and S31603 general-purpose stainless steel was used for piping other than those. Anticorrosive air was mixed in advance into raw material carbon dioxide such that an oxygen feed concentration with respect to the carbon dioxide was about 1000 ppm in a carbon dioxide compression step. A pressure in the urea synthesis system was 136 bar.

1237 t/d of raw material ammonia is fed from a line 1, and 1600 t/d of the raw material carbon dioxide is fed to a compressor from a line 14. The raw material ammonia was pressurized to 180 bar by a pump, heated to 147°C with steam condensate in a heat exchanger D from the line 1, and fed to a bottom of the urea synthesis tower A through the ejector G. Further, 1528 t/d of the raw material carbon dioxide and about 14 t/d of the anticorrosive air were pressurized to 141 bar by the compressor, and 1233 t/d of the raw material carbon dioxide and about 11 t/d of the anticorrosive air were fed to a bottom of the stripper C via lines 2 and 2b. 295 t/d of the carbon dioxide and about 3 t/d of the anticorrosive air were fed to the bottom of the urea synthesis tower A via a line 2a (ratio [X_{C}/X_{A}] = about 81/19). Further, 72 t/d of the raw material carbon dioxide was branched from an intermediate stage of the compressor and fed via a line 13 to a low-pressure decomposition tower in a decomposition step downstream from the stripper. On the other hand, 1849 t/d of a recovered liquid as an absorbing medium from a line 7 has the composition below, and is pressurized to 141 bar at a temperature of 106°C and fed to a scrubber F.

Urea 0.2%, ammonia 35.0%, carbon dioxide 40.6%, water 24.2%

The scrubber F was operated at 136 bar and 160°C. An outlet liquid of the scrubber F was fed to a bottom of the condenser B via a receiver 16 and a downpipe 11. On the other hand, inert gases were released from a tower top of the scrubber F via a line 15.

A mixed gas composed of ammonia, carbon dioxide, water and inert gases separated in the stripper C was sent to the bottom of the condenser B via a line 5. A shellside of the condenser B is filled with the recovered liquid and ammonium carbamate produced through condensation, and the mixed gas is absorbed by this liquid. Note that absorption heat is subjected to heat removal by a cooler indicated with lines 13 and 14. After a 20-minute residence time, condensate that absorbed the mixed gas flowed down through a downpipe 3 by gravity, and was fed to the bottom of the urea synthesis tower A after pressurized in the ejector G.

The liquid raw material ammonia and the condensate fed to the bottom of the urea synthesis tower A further promoted the urea synthesis reaction while they ascended in the urea synthesis tower A. A residence time then was 20 minutes.

A synthesis liquid fed from a top of the urea synthesis tower A to the stripper C via a line 4 is subjected to thermal decomposition of ammonium carbamate and separation of ammonia and carbon dioxide as the mixed gas. The stripper C was operated at a tower top temperature of 180°C and a top pressure of 136 bar.

As described above, gases and liquids were circulated between the condenser B, the urea synthesis tower A and the stripper C to perform operation. The ejector G used as driving force for that using the liquid raw material ammonia as a driving fluid had a differential pressure of about 3 bar between a discharge side and a suction side.

Table 1 shows power of the pumps for pressurizing the raw material ammonia and the recovered liquid, and power of the compressor for pressurizing the raw material carbon dioxide, Tables 2 to 5 show operating conditions of the urea synthesis tower A, the condenser B, the stripper C and the ejector G, and Table 6 shows composition at a tower bottom (line 12).

### <Reference example 1>

Urea synthesis was performed by a urea synthesis process using the urea synthesis apparatus with a daily production capacity of 2200 tons shown in FIG. 2, in which S31603 general-purpose stainless steel was used for the entire piping connecting devices with one another.

Anticorrosive air was mixed in advance into raw material carbon dioxide such that an oxygen concentration with respect to the carbon dioxide was 5000 ppm in a carbon dioxide compression step to prevent corrosion of S31603 general-purpose stainless steel used for the piping.

In order to suppress a flow rate of process gases (ammonia, carbon dioxide and water) entrained in inert gases flowing from the tower top of the urea synthesis tower A and the tower top of the condenser B to the scrubber F, an overpressure, a difference between an operating pressure and a saturated vapor pressure of the liquid was increased, and an operating pressure of the synthesis system was 152 bar.

Table 1 shows power of the pumps for pressurizing the raw material ammonia and the recovered liquid, and power of the compressor for pressurizing the raw material carbon dioxide, Tables 2 to 5 show operating conditions of the urea synthesis tower A, the condenser B, the stripper C and the ejector G, and Table 6 shows composition at a tower bottom (line 12).

### [Table 1]

**Table 1**

| | | | Example 1 | Reference example 1 |
|---|---|---|---|---|
| Feed amount of raw material ammonia (t/d) | | | 1237 | 1237 |
| Feed amount of raw material carbon dioxide (t/d) | | | 1600 | 1600 |
| Recovered liquid | Amount of liquid (t/d) | | 1849 | 1902 |
| | Composition (%) | Urea | 0.2 | 0.2 |
| | | Ammonia | 35 | 35.3 |
| | | Carbon dioxide | 40.6 | 40.2 |
| | | Water | 24.2 | 24.3 |
| Pump for pressurizing raw material ammonia | | Discharge pressure (bar) | 180 | 196 |
| | | Power (kW) | 716 | 783 |
| Pump for pressurizing recovered liquid | | Discharge pressure (bar) | 141 | 157 |
| | | Power (kW) | 439 | 507 |
| Compressor for pressurizing raw material carbon dioxide | | Discharge pressure (bar) | 141 | 157 |
| | | Power (kW) | 8486 | 8655 |

**[Table 2]**

| Table 2-Urea synthesis tower A | | |
|---|---|---|
| | Example 1 | Reference example 1 |
| Pressure (bar) | 136 | 152 |
| Liquid N/C | 3.7 | 3.7 |
| Liquid H/C | 0.56 | 0.58 |
| Residence time (minute) | 20 | 20 |

**[Table 3]**

| Table 3-Condenser B | | |
|---|---|---|
| | Example 1 | Reference example 1 |
| Tower top pressure (bar) | 136 | 152 |
| Liquid N/C | 3 | 2.9 |
| Liquid H/C | 0.63 | 0.65 |
| Residence time (minute) | 20 | 20 |

**[Table 4]**

| Table 4-Stripper C | | |
|---|---|---|
| | Example 1 | Reference example 1 |
| Tower top pressure (bar) | 136 | 152 |
| Pressure of heating steam (bar) | 18.1 | 19.6 |

**[Table 5]**

| Table 5-Ejector G | | |
|---|---|---|
| | Example 1 | Reference example 1 |
| Suction side pressure (bar) | 136 | 152 |
| Discharge side pressure (bar) | 139 | 155 |

**[Table 6]**

| Table 6-Tower bottom (line 12) | | |
|---|---|---|
| Composition (%) | Example 1 | Reference example 1 |
| Urea | 50.3 | 49.5 |
| Ammonia | 12.2 | 12.9 |
| Carbon dioxide | 12.5 | 12.7 |
| Water | 25 | 24.9 |

### <Evaluation>

In example 1, due to the reduced operating pressure of the synthesis system, the total power of the pumps for pressurizing ammonia and the recovered liquid was reduced by 135 kW (about 10%) and the power of the compressor for the raw material carbon dioxide was reduced by 169 kW (about 2%) compared to reference example 1.

Further, separation efficiency in the stripper is improved due to the low pressure, and thus, the concentrations of ammonia and carbon dioxide obtained at the tower bottom of the stripper are lower even if heating steam at a low pressure of 18.1 bar is used. The low concentrations result in less energy for separating ammonia and carbon dioxide in a separation step (not shown in FIG. 2) downstream from the stripper. Further, the recovered liquid recovered as an aqueous solution of separated ammonia and water is also reduced in amount, and less amount of water is brought into the synthesis system, resulting in a reduced H/C in the synthesis system, and the low H/C contributes to an improved conversion rate of carbon dioxide.

### Industrial Applicability

The present invention is very useful as a method for synthesizing urea from ammonia and carbon dioxide while forming a passivation film with oxygen on a metal surface inside an apparatus, particularly as a urea synthesis method capable of reducing energy or running costs required for urea synthesis.

### Reference Signs List

- A: urea synthesis tower
- B: condenser
- C: stripper
- D: heat exchanger
- F: scrubber
- G: ejector
- H: carbonic acid gas compressor
- I: recovery system
- 1, 2, 2a, 2b, 4, 5, 7, 12, 13, 14, 15: line
- 3, 11: downpipe
- 16: receiver

## Claims

1. A urea synthesis method comprising, reacting ammonia with carbon dioxide at a urea synthesis temperature and a urea synthesis pressure in a urea synthesis tower, bringing a urea synthesis liquid containing at least produced urea, unreacted ammonia, unreacted carbon dioxide and water into contact with at least part of the raw material carbon dioxide under heating at a pressure substantially equal to the urea synthesis pressure in a stripper to separate the unreacted ammonia and the unreacted carbon dioxide as a mixed gas of ammonia, carbon dioxide and water,
further treating the urea synthesis liquid containing unseparated unreacted ammonia and unreacted carbon dioxide to obtain urea, while introducing the mixed gas separated in the stripper into a bottom of a condenser to bring the mixed gas into contact with an absorbing medium under cooling to thereby condense the mixed gas, and circulating the thus obtained condensate to the urea synthesis tower, **characterized in that**
an oxygen feed concentration with respect to the raw material carbon dioxide is 100 to 2,000 ppm,
the urea synthesis pressure in the urea synthesis tower is 125 to 145 bar,
the urea synthesis temperature in the urea synthesis tower is 175°C to 190°C,
a N/C (molar ratio of ammonia to carbon dioxide) in the urea synthesis tower is 3.5 to 4.0, and
a H/C (molar ratio of water to carbon dioxide) in the urea synthesis tower is 0.70 or less.

2. The urea synthesis method according to Claim 1, wherein a N/C (molar ratio of ammonia to carbon dioxide) in the condenser is 2.7 to 3.3.

3. The urea synthesis method according to Claim 1, wherein a residence time in the urea synthesis tower is 10 to 40 minutes.

4. The urea synthesis method according to Claim 1, wherein most of the raw material carbon dioxide with the oxygen concentration of 100 to 2,000 ppm is fed to the stripper, and the remainder thereof is fed to the urea synthesis tower.

5. The urea synthesis method according to Claim 1, wherein a material of at least one device selected from the group consisting of the urea synthesis tower, the stripper, the condenser, and piping connecting at least two of the urea synthesis tower, the stripper and the condenser is
(a) a general-purpose austenitic-ferritic duplex stainless steel with a Cr content of 21 to 26 mass%, a Ni content of 4.5 to 7.5 mass%, a Mo content of 2.5 to 3.5 mass%, a N content of 0.08 to 0.30 mass%, a C content of 0.03 mass% or less, a Si content of 1.0 mass% or less, a Mn content of 2.0 mass% or less, a P content of 0.04 mass% or less, and a S content of 0.03 mass% or less, or
(b) an austenitic-ferritic duplex stainless steel with a Cr content of 26 to 35 mass%, a Ni content of 3 to 10 mass%, a Mo content of 1.0 to 4.0 mass%, and a N content of 0.2 to 0.6 mass%.

6. The urea synthesis method according to Claim 1, wherein a material of the stripper is
(a) a general-purpose austenitic-ferritic duplex stainless steel with a Cr content of 21 to 26 mass%, a Ni content of 4.5 to 7.5 mass%, a Mo content of 2.5 to 3.5 mass%, a N content of 0.08 to 0.30 mass%, a C content of 0.03 mass% or less, a Si content of 1.0 mass% or less, a Mn content of 2.0 mass% or less, a P content of 0.04 mass% or less, and a S content of 0.03 mass% or less, or
(b) an austenitic-ferritic duplex stainless steel with a Cr content of 26 to 35 mass%, a Ni content of 3 to 10 mass%, a Mo content of 1.0 to 4.0 mass%, and a N content of 0.2 to 0.6 mass%.

7. The urea synthesis method according to Claim 1, wherein a material of piping connecting the stripper and the condenser is
(a) a general-purpose austenitic-ferritic duplex stainless steel with a Cr content of 21 to 26 mass%, a Ni content of 4.5 to 7.5 mass%, a Mo content of 2.5 to 3.5 mass%, a N content of 0.08 to 0.30 mass%, a C content of 0.03 mass% or less, a Si content of 1.0 mass% or less, a Mn content of 2.0 mass% or less, a P content of 0.04 mass% or less, and a S content of 0.03 mass% or less, or
(b) an austenitic-ferritic duplex stainless steel with a Cr content of 26 to 35 mass%, a Ni content of 3 to 10 mass%, a Mo content of 1.0 to 4.0 mass%, and a N content of 0.2 to 0.6 mass%.

8. A method for improving an existing urea synthesis apparatus to reduce a urea synthesis pressure in a urea synthesis tower, wherein
the existing urea synthesis apparatus is a urea synthesis apparatus for reacting ammonia with carbon dioxide at a urea synthesis temperature and a urea synthesis pressure in a urea synthesis tower, bringing a urea synthesis liquid containing at least produced urea, unreacted ammonia, unreacted carbon dioxide and water into contact with at least part of the raw material carbon dioxide under heating at a pressure substantially equal to the urea synthesis pressure in a stripper to separate the unreacted ammonia and the unreacted carbon dioxide as a mixed gas of ammonia, carbon dioxide and water, further treating the urea synthesis liquid containing unseparated unreacted ammonia and unreacted carbon dioxide to obtain urea, while introducing the mixed gas separated in the stripper into a bottom of a condenser to bring the mixed gas into contact with an absorbing medium under cooling to thereby condense the mixed gas, and circulating the thus obtained condensate to the urea synthesis tower, and
a material of piping connecting the stripper and the condenser in the existing urea synthesis apparatus is neither of the materials (a) and (b) below,
the method including, replacing the piping connecting the stripper and the condenser in the existing urea synthesis apparatus with piping including the material (a) or (b) below to enable reduction of the urea synthesis pressure in the urea synthesis tower,
(a) a general-purpose austenitic-ferritic duplex stainless steel with a Cr content of 21 to 26 mass%, a Ni content of 4.5 to 7.5 mass%, a Mo content of 2.5 to 3.5 mass%, a N content of 0.08 to 0.30 mass%, a C content of 0.03 mass% or less, a Si content of 1.0 mass% or less, a Mn content of 2.0 mass% or less, a P content of 0.04 mass% or less, and a S content of 0.03 mass% or less, and
(b) an austenitic-ferritic duplex stainless steel with a Cr content of 26 to 35 mass%, a Ni content of 3 to 10 mass%, a Mo content of 1.0 to 4.0 mass%, and a N content of 0.2 to 0.6 mass%.

9. A method for improving an existing urea synthesis apparatus to reduce a urea synthesis pressure in a urea synthesis zone, wherein
the existing urea synthesis apparatus is a urea synthesis apparatus for reacting ammonia with carbon dioxide at a urea synthesis temperature and a urea synthesis pressure in a urea synthesis zone, bringing a urea synthesis liquid containing at least produced urea, unreacted ammonia, unreacted carbon dioxide and water into contact with at least part of the raw material carbon dioxide under heating at a pressure substantially equal to the urea synthesis pressure in a stripper to separate the unreacted ammonia and the unreacted carbon dioxide as a mixed gas of ammonia, carbon dioxide and water, further treating the urea synthesis liquid containing unseparated unreacted ammonia and unreacted carbon dioxide to obtain urea, while introducing the mixed gas separated in the stripper into a bottom of a condensation zone to bring the mixed gas into contact with an absorbing medium under cooling to thereby condense the mixed gas, and circulating the thus obtained condensate to the urea synthesis zone,
the urea synthesis zone and the condensation zone are present in the same container or in separate containers,
the condensation zone is present in a horizontal container, and
a material of piping connecting the stripper and the condensation zone in the existing urea synthesis apparatus is neither of the materials (a) and (b) below,
the method including, replacing the piping connecting the stripper and the condensation zone in the existing urea synthesis apparatus with piping including the material (a) or (b) below to enable reduction of the urea synthesis pressure in the urea synthesis zone,
(a) a general-purpose austenitic-ferritic duplex stainless steel with a Cr content of 21 to 26 mass%, a Ni content of 4.5 to 7.5 mass%, a Mo content of 2.5 to 3.5 mass%, a N content of 0.08 to 0.30 mass%, a C content of 0.03 mass% or less, a Si content of 1.0 mass% or less, a Mn content of 2.0 mass% or less, a P content of 0.04 mass% or less, and a S content of 0.03 mass% or less, and
(b) an austenitic-ferritic duplex stainless steel with a Cr content of 26 to 35 mass%, a Ni content of 3 to 10 mass%, a Mo content of 1.0 to 4.0 mass%, and a N content of 0.2 to 0.6 mass%.
